(19) Europäisches Patentamt / European Patent Office / Office européen des brevets

(11) **EP 4 404 138 B1**

(12) **EUROPÄISCHE PATENTSCHRIFT**

(45) Veröffentlichungstag und Bekanntmachung des Hinweises auf die Patenterteilung:
**09.04.2025 Patentblatt 2025/15**

(21) Anmeldenummer: **23151982.8**

(22) Anmeldetag: **17.01.2023**

(51) Internationale Patentklassifikation (IPC):
**G06T 7/30** (2017.01)   **G06T 11/00** (2006.01)
**A61B 6/00** (2024.01)

(52) Gemeinsame Patentklassifikation (CPC):
**G06T 11/005; A61B 6/5264; G06T 7/30;**
G06T 2207/10076; G06T 2207/20084;
G06T 2211/412; G06T 2211/441

(54) **BEWEGUNGSKOMPENSIERTE BILDREKONSTRUKTION BEI EINEM RÖNTGENBASIERTEN BILDGEBUNGSVERFAHREN**

MOTION COMPENSATED IMAGE RECONSTRUCTION IN AN X-RAY BASED IMAGING METHOD

RECONSTRUCTION D'IMAGE À COMPENSATION DE MOUVEMENT DANS UN PROCÉDÉ D'IMAGERIE À RAYONS X

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**24.07.2024 Patentblatt 2024/30**

(73) Patentinhaber: **Siemens Healthineers AG**
**91301 Forchheim (DE)**

(72) Erfinder:
• **Manhart, Michael**
**90765 Fürth (DE)**
• **Meier, Manuela**
**90425 Nürnberg (DE)**
• **Preuhs, Alexander**
**91058 Erlangen (DE)**

(74) Vertreter: **Siemens Healthineers**
**Patent Attorneys**
**Postfach 22 16 34**
**80506 München (DE)**

(56) Entgegenhaltungen:
• **MEZHERITSKY TAL ET AL: "Population-based 3D respiratory motion modelling from convolutional autoencoders for 2D ultrasound-guided radiotherapy", MEDICAL IMAGE ANALYSIS, OXFORD UNIVERSITY PRESS, OXOFRD, GB, vol. 75, 9 October 2021 (2021-10-09), XP086895357, ISSN: 1361-8415, [retrieved on 20211009], DOI: 10.1016/ J.MEDIA.2021.102260**
• **V. SITZMANNJ. MARTELA. BERGMANA. LINDELLG. WETZSTEIN: "Implicit neural representations with periodic activation functions", ADVANCES IN NEURAL INFORMATION PROCESSING SYSTEMS., vol. 33, 2020, pages 7462 - 73, XP093055806**

**Beschreibung**

Die vorliegende Erfindung ist auf ein Verfahren zur Erzeugung einer bewegungskompensierten Bildrekonstruktion bei einem röntgenbasierten Bildgebungsverfahren gerichtet, wobei eine Vielzahl während der Durchführung des Bildgebungsverfahrens erzeugter Projektionsbilder eines Objekts bereitgestellt wird. Die Erfindung ist ferner auf ein Verfahren zur röntgenbasierten Bildgebung gerichtet, wobei eine Vielzahl von Projektionsbildern eines Objekts erzeugt wird und ein Verfahren zur Erzeugung einer bewegungskompensierten Bildrekonstruktion durchgeführt wird. Die Erfindung ist außerdem auf eine Datenverarbeitungsvorrichtung, eine Vorrichtung zur röntgenbasierten Bildgebung aufweisend eine Röntgenbildgebungsmodalität und ein Computerprogrammprodukt gerichtet.

Mit Hilfe röntgenbasierter Bildgebungsverfahren, insbesondere der Kegelstrahl-Computertomographie, CBCT (englisch: "cone-beam computed tomography"), lassen sich unter anderem Gehirnstrukturen während chirurgischer Eingriffe beurteilen. Dies hilft zum Beispiel bei der Differenzialdiagnose von Schlaganfällen zur Zuordnung zur ischämischen oder der hämorrhagischen Kategorie. Ein wesentliches Hindernis für eine qualitativ hochwertige Bildbeurteilung ist jedoch die Bewegung des Patienten, die die für die Rekonstruktion verwendeten geometrischen Informationen verfälscht. Dies führt zu Streifen- oder Unschärfe-Artefakten.

Es können Techniken zur Bewegungskompensation eingesetzt werden, um die Bewegung retrospektiv zu korrigieren. Dazu wird eine starre Bewegungstrajektorie des Patienten geschätzt. Bei erfolgreicher Schätzung der starren Bewegungstrajektorie kann eine bewegungskompensierte Bildrekonstruktion erstellt werden.

Dabei wird die Bewegung insbesondere durch Optimierung einer Zielfunktion kompensiert. Die Eingabe für die Zielfunktion enthält einen Satz von Projektionsbildern und ein Satz von Parametern, die die approximierte Bewegungstrajektorie des Patienten definieren. Werden die Projektionsbilder als I bezeichnet und die Parameter, welche die Bewegungstrajektorie definieren, als X bezeichnet, dann kann das Problem der Bewegungskompensation durch die Bestimmung optimaler Parameter X' wie folgt gelöst werden

$$X' = argmin_X c\big(r(I, X)\big),$$

wobei r() die Bildrekonstruktion auf der Grundlage der Projektionsbilder und der gegenwärtigen Schätzung der Bewegungstrajektorie bezeichnet und c() eine Kostenfunktion bezeichnet. Die Kostenfunktion ist beispielsweise mit der Bewegung korreliert, sodass niedrige Werte einer geringen Bewegung entsprechen.

Wie in der Publikation J.Wang, R. Schaffert, A. Borsdorf, B. Heigl, X. Huang, J. Hornegger, A. Maier: "Dynamic 2-D/3-D rigid registration framework using point-to-plane correspondence model", IEEE Transactions on Medical Imaging, Vol. 36, No. 9, pp. 1939-1954, 2017 beschrieben, kann die Kostenfunktion beispielsweise auf einer 2D/3D-Bildregistrierung basieren.

Die Kostenfunktion kann auch auf Konsistenzbedingungen basieren, wie in den Publikationen A. Preuhs, M. Manhart, A. Maier: "Fast Epipolar Consistency without the Need for Pseudo Matrix Inverses", Proc. 15th International Conference on Image Formation in X-Ray Computed Tomography, pp. 202-205, 2018 und A. Preuhs, M. Manhart, E. Hoppe, M. Kowarschik, A. Maier: "Motion gradients for epipolar consistency", Proc. 15th International Meeting on Fully Three-Dimensional Image Reconstruction in Radiology and Nuclear Medicine, p. 110720D, 2019 beschrieben. In diesem Fall ist in der obigen Gleichung die Bildrekonstruktion r() nicht erforderlich.

Ferner kann die Kostenfunktion auf Autofokusansätzen beruhen, wie in der Publikation A. Preuhs, M. Manhart, P. Roser, E. Hoppe, H. Yixing, M. Psychogios, M. Kowarschik, A. Maier: "Appearance Learning for Image-based Motion Estimation in Tomography", IEEE Transactions on Medical Imaging, pp. 1-10, 2020 beschrieben.

Eine mögliche Parametrisierung X basiert auf Rotationen und Translationen. In diesem Fall werden für jedes Projektionsbild drei Parameter für die Rotation und drei Parameter für die Translation verwendet. Ist die Anzahl der Projektionsbilder N, so beträgt die Anzahl der benötigten Parameter N*6. Für eine klassische CBCT-Trajektorie, die zum Beispiel 400-600 Projektionsbilder umfasst, hätte der Parametervektor X 2400-3600 Einträge. Dies macht das Optimierungsproblem sehr rechenintensiv.

Um dieses Problem zu entschärfen, können Splines zur Bewegungskompensation verwendet werden, wie in der oben genannten Publikation von Preuhs et al. aus 2020 beschrieben. In diesem Fall wird die Bewegung durch Spline-Knoten parametrisiert. Dadurch lässt sich die Komplexität des Bewegungskompensationsproblems stark reduzieren. So kann beispielsweise eine einzelne Bewegungsrichtung durch 20 Spline-Knoten approximiert werden, was zu einem Parametervektor X mit nur 120 Einträgen führt. Ein weiterer Vorteil des Splines ist die Einbeziehung von Beschränkungen. So ist die Bewegung des Patienten zum Beispiel in der Regel gleichmäßig, was durch Splines inhärent berücksichtigt wird.

Ein Problem bei beiden genannten Parametrisierungen ist, dass sie viele Bewegungstrajektorien ermöglichen, die physikalisch nicht sinnvoll sind. Da die Kostenfunktion in der Regel nicht konvex ist, können diese physikalisch nicht sinnvollen Bewegungstrajektorien die Optimierung in lokale Minima führen und die erfolgreiche Konvergenz verhindern. Dadurch wird die zuverlässige Bewegungskompensation zum Teil verhindert.

Es ist eine Aufgabe der vorliegenden Erfindung, die Zuverlässigkeit der Bewegungskompensation bei der Bildrekonstruktion bei röntgenbasierten Bildgebungsverfahren zu erhöhen.

Diese Aufgabe wird durch den jeweiligen Gegenstand der unabhängigen Ansprüche gelöst. Vorteilhafte Weiterbildungen und bevorzugte Ausführungsformen sind Gegenstand der abhängigen Ansprüche. Unabhängig vom grammatikalischen Geschlecht eines bestimmten Begriffes sind Personen mit männlicher, weiblicher oder anderer Geschlechteridentität mit umfasst.

Die Erfindung beruht auf dem Gedanken, eine Parametrisierung einer Objektbewegungstrajektorie derart zu wählen, dass unrealistische oder physikalisch unmögliche Bewegungskonfigurationen bei der Optimierung nicht berücksichtigt werden. Dazu werden die Objektbewegungstrajektorien in einem abstrakten latenten Raum durch entsprechende latente Vektoren parametrisiert und konkrete Objektbewegungstrajektorien durch Anwendung eines entsprechend trainierten Algorithmus erzeugt.

Gemäß einem Aspekt der Erfindung wird ein Verfahren zur Erzeugung einer bewegungskompensierten Bildrekonstruktion bei einem röntgenbasierten Bildgebungsverfahren angegeben. Dabei werden die folgenden Schritte i), ii), iii) und iv) durchgeführt, jedoch nicht notwendiger Weise in dieser Reihenfolge. Gemäß Schritt i) wird eine Vielzahl während der Durchführung des Bildgebungsverfahrens erzeugter Projektionsbilder eines Objekts bereitgestellt. Gemäß ii) wird ein trainierter Algorithmus bereitgestellt, insbesondere auf einem Speichergerät gespeichert, der dazu trainiert ist, einem latenten Vektor eines vorgegebenen latenten Raums eine Objektbewegungstrajektorie zuzuweisen. Gemäß Schritt iii) wird eine optimale Objektbewegungstrajektorie durch Anwendung des trainierten Algorithmus auf einen optimalen latenten Vektor, insbesondere einen optimalen latenten Vektor des latenten Raums, bestimmt und die bewegungskompensierte Bildrekonstruktion wird abhängig von der Vielzahl von Projektionsbildern unter der Annahme erzeugt, das Objekt habe sich während der Durchführung des Bildgebungsverfahrens gemäß der optimalen Objektbewegungstrajektorie bewegt.

Gemäß Schritt iv) wird der optimale latente Vektor bestimmt, indem eine vorgegebene Kostenfunktion unter Verwendung des latenten Vektors als Optimierungsparameter minimiert wird. Für jeden Optimierungsschritt mit einem gegenwärtigen latenten Vektor wird dabei durch Anwendung des trainierten Algorithmus auf dem gegenwärtigen latenten Vektor eine gegenwärtige Objektbewegungstrajektorie bestimmt. Ein gegenwärtiger Wert der Kostenfunktion wird abhängig von der Vielzahl von Projektionsbildern unter der Annahme berechnet, das Objekt habe sich während der Durchführung des Bildgebungsverfahrens gemäß der gegenwärtigen Objektbewegungstrajektorie bewegt.

Je nach eingesetzter Kostenfunktion kann dabei für jeden der Optimierungsschritte beispielsweise der gegenwärtige Wert der Kostenfunktion abhängig von einem vorgegebenen Satz von Konsistenzbedingungen, insbesondere epipolaren Konsistenzbedingungen, betreffend die Vielzahl von Projektionsbildern berechnet werden.

Dies ist beispielsweise bei der Verwendung einer Kostenfunktion wie in den eingangs genannten Publikationen von Preuhs et al. aus 2018 und 2019 beschrieben der Fall.

Alternativ kann für jeden der Optimierungsschritte beispielsweise eine gegenwärtige Bildrekonstruktion abhängig von der Vielzahl von Projektionsbildern unter der Annahme erzeugt werden, das Objekt habe sich während der Durchführung des Bildgebungsverfahrens gemäß der gegenwärtigen Objektbewegungstrajektorie bewegt. Der gegenwärtige Wert der Kostenfunktion wird dann abhängig von der gegenwärtigen Bildrekonstruktion berechnet.

Dies ist beispielsweise bei der Verwendung von Kostenfunktionen wie in der eingangs genannten Publikation von Wang et al. oder der eingangs genannten Publikation von Preuhs et al. aus 2020 beschrieben der Fall.

Das erfindungsgemäße Verfahren zur Erzeugung einer bewegungskompensierten Bildrekonstruktion kann in einigen Ausführungsformen ein rein computerimplementiertes Verfahren sein, bei dem alle Schritte des computerimplementierten Verfahrens von einer Datenverarbeitungsvorrichtung durchgeführt werden, welche wenigstens eine Recheneinheit aufweist. Insbesondere ist die wenigstens eine Recheneinheit zur Durchführung der Schritte des computerimplementierten Verfahrens konfiguriert oder angepasst. Hierzu kann die wenigstens eine Recheneinheit beziehungsweise ein Speichergerät der wenigstens einen Recheneinheit beispielsweise ein Computerprogramm speichern, welches Befehle beinhaltet, die bei Ausführung durch die wenigstens eine Recheneinheit die wenigstens eine Recheneinheit dazu veranlassen, das computerimplementierte Verfahren auszuführen.

In anderen Ausführungsformen ist das Verfahren nicht rein computerimplementiert, beispielsweise wenn der Schritt der Durchführung des Bildgebungsverfahrens, also insbesondere des Erzeugens der Projektionsbilder, Teil des Verfahrens ist.

Das röntgenbasierte Bildgebungsverfahren kann beispielsweise ein Computertomographieverfahren, insbesondere ein Kegelstrahl-Computertomographieverfahren sein. Die Projektionsbilder entsprechen insbesondere mittels eines Röntgendetektors detektierter Röntgenstrahlung, die durch das Objekt hindurchgetreten und dementsprechend abgeschwächt sind. Das Erzeugen einer Bildrekonstruktion basierend auf solchen Projektionsbildern beinhaltet dann insbesondere die Bestimmung entsprechender Abschwächungswerte in den zugehörigen räumlichen Bereichen des Objekts.

Zur Bildrekonstruktion können verschiedene bekannte Verfahren verwendet werden. Insbesondere kann zur Bildrekonstruktion jedem der Projektionsbilder eine geometrische Operation zugewiesen sein, mittels der aus dem jeweiligen Projektionsbild die Bildrekonstruktion beziehungsweise ein Teil der Bildrekonstruktion erzeugt wird. Ebenfalls ist es bekannt, bei solchen Bildrekonstruktionen eine Bewegungskompensation durchzuführen, um Bewegungen des Objekts, insbesondere starre Bewegungen des Objekts, während der Aufnahme der Projektionsbilder zu kompensieren. Hierzu werden die den einzelnen Projektionsbildern zugeordneten geometrischen Operationen entsprechend der jeweils geschätzten oder angenommenen Objektbewegungstrajektorie beziehungsweise der entsprechenden Translations- und Rotationsposition des Objekts bei der Erzeugung des entsprechenden Projektionsbilds, korrigiert.

Diese Korrektur kann beispielsweise durch entsprechende Rotationen und/oder Translationen erfolgen, die gemäß der Objektbewegungstrajektorie zum entsprechenden Zeitpunkt der Erzeugung des Projektionsbilds vorliegen. Mit anderen Worten kann eine Objektbewegungstrajektorie durch eine Anzahl aufeinanderfolgender Rotationsoperationen und/oder Translationsoperationen definiert sein. Das Format, in dem eine Objektbewegungstrajektorie angegeben oder gespeichert wird, kann dabei verschieden sein. Es können entsprechende Translationsvektoren und Rotationsmatrizen angegeben werden oder entsprechende Rotationswinkel und Translationswerte und so weiter. Um eine Translation und Rotation für ein gegebenes Projektionsbild, also einen zugehörigen Schritt der Objektbewegungstrajektorie, festzulegen, sind dementsprechend im Allgemeinen drei Rotationsparameter und drei Translationsparameter erforderlich. Die Objektbewegungstrajektorie beinhaltet daher für jedes Projektionsbild diese drei Rotationsparameter und Translationsparameter direkt oder in indirekter Form.

Der latente Vektor kann beispielsweise als Tupel mit N Einträgen verstanden werden, wobei die Einträge reellen oder komplexen Zahlen entsprechen können. Die einzelnen Einträge des latenten Vektors haben im Allgemeinen keine anschauliche Bedeutung. Der trainierte Algorithmus ist dazu trainiert, aus jedem beliebigen latenten Vektor des latenten Raums genau eine Objektbewegungstrajektorie zu berechnen, also eine Sequenz von Rotations- und/oder Translationsoperationen für jedes der Projektionsbilder oder eine Sequenz der entsprechenden Rotationsparameter und Translationsparameter für die einzelnen Projektionsbilder.

Die Größe von N kann an die erforderliche Genauigkeit und die zur Verfügung stehenden Rechenressourcen angepasst werden. Typischerweise können latente Räume mit 50 bis 150 Dimensionen, also dementsprechend latente Vektoren mit 50 bis 150 Einträgen, verwendet werden. Auf diese Weise wird erreicht, dass eine ausreichende Modifikation der latenten Vektoren möglich ist, um komplexe Objekte wie die Objektbewegungstrajektorie codieren zu können. Der latente Raum kann beispielsweise $\mathbb{R}^N$

oder $\mathbb{C}^N$ sein.

Es ist bekannt, komplexe Eingaben, wie etwa Texte, Bilder und so weiter eindeutig in einen latenten Raum zu codieren, indem durch maschinelles Lernen trainierte Algorithmen eingesetzt werden. Hierfür ist es insbesondere bekannt, künstliche neuronale Netzwerke einzusetzen, beispielsweise sogenannte Autoencoder-Netzwerke oder Variational Autoencoder Netzwerke. Soweit nicht anders angegeben kann im Folgenden die Bezeichnung Autoencoder derart verstanden werden, dass Autoencoder im engeren Sinne ebenso wie Variational Autoencoder umfasst ist. Statt Autoencoder Netzwerken können beispielsweise auch generative adverse Netzwerke, GAN (englisch: "Generative Adversarial Networks") eingesetzt werden.

Solche Algorithmen können in einer selbstüberwachten Weise trainiert werden, sodass sie einerseits aus einer komplexen Eingabe genau einen latenten Vektor erzeugen können und aus demselben latenten Vektor die Eingabe rekonstruieren können. Ist der Algorithmus einmal trainiert, so kann der rekonstruierende Teil des Algorithmus, je nach Ausgestaltungsform auch als Generatormodul oder Decodermodul bezeichnet, isoliert werden und zur Erzeugung der rekonstruierten Eingabe basierend auf beliebigen latenten Vektoren verwendet werden.

Dieses Konzept wird erfindungsgemäß zur Erzeugung von Objektbewegungstrajektorien aus latenten Vektoren genutzt. Der trainierte Algorithmus wurde also, insbesondere unter Zugrundelegung ausschließlich physikalisch möglicher Trainingsobjektbewegungstrajektorien, trainiert, sodass er nach dem Training jeder solchen Trainingsobjektbewegungstrajektorie genau einen latenten Vektor zuweisen kann und aus diesem latenten Vektor zuverlässig und akkurat die Trainingsobjektbewegungstrajektorie rekonstruieren kann. Um sicherzustellen, dass nur physikalisch mögliche Trainingsobjektbewegungstrajektorien zugrunde gelegt werden und dementsprechend die Rekonstruktion eines beliebigen latenten Vektors ebenfalls physikalisch möglich ist, können die Trainingsobjektbewegungstrajektorien beispielsweise aus der Überwachung der tatsächlichen Bewegung von Patienten gewonnen werden.

Für das erfindungsgemäße Verfahren zur Erzeugung der bewegungskompensierten Bildrekonstruktion wird lediglich der rekonstruierende Anteil benötigt. Die latenten Vektoren, die während der Optimierung als gegenwärtige latente Vektoren als Eingabe dem trainierten Algorithmus zugrundegelegt werden, sind in dem vorgegebenen latenten Raum beliebig. Dementsprechend resultieren auch beliebige, jedoch stets physikalisch mögliche, gegenwärtige Objektbewegungstrajektorien.

Durch die Rekonstruktion und Auswertung der Kostenfunktion wird derjenige latente Vektor als optimaler latenter Vektor identifiziert, der die Kostenfunktion minimiert. Die Kostenfunktion ist dabei wie eingangs beschrieben mit der tatsächlichen Objektbewegung korreliert, sodass niedrige Werte der Kostenfunktion einem geringen Maß an Bewegung entsprechen. Dementsprechend wird diejenige Objektbewegungstrajektorie als optimale Objektbewegungstrajektorie identifiziert, die einer möglichst realistischen, da geringen, Bewegung des Objekts während der Durchführung des röntgenbasierten Bildgebungsverfahrens entsprechen.

Lediglich zur Klarstellung sei erwähnt, dass das Verwenden des latenten Vektors als Optimierungsparameter derart verstanden werden kann, dass für unterschiedliche Optimierungsschritte unterschiedliche gegenwärtige latente Vektoren aus dem latenten Raum ausgewählt werden, um den entsprechenden Optimierungsschritt durchzuführen. Als Optimierungsverfahren können ebenfalls verschiedene bekannte Optimierungsverfahren eingesetzt werden.

In Optimierungsproblemen wird allgemein von Zielfunktionen gesprochen, unabhängig davon, ob diese zu minimieren sind oder zu maximieren. Wenn die Zielfunktion zu minimieren sind, handelt es sich um eine Kostenfunktion. Dabei sei darauf hingewiesen, dass die Minimierung jedenfalls im vorliegenden Fall keinerlei Einschränkung darstellt, da auch im Falle **ei**ner zu maximierenden Zielfunktion stets eine modifizierte Zielfunktion definiert werden kann, welche es zu minimieren gilt.

In dem erfindungsgemäßen Verfahren zur Erzeugung einer bewegungskompensierten Bildrekonstruktion wird die Erkenntnis ausgenutzt, dass es sich bei dem Bewegungskompensationsproblem um ein nicht-konvexes Problem handelt, indem lokale Minima in der Optimierung problematisch werden können, wenn diese physikalisch nicht sinnvollen Bewegungskonfigurationen entsprechen. Durch die Parametrisierung anhand der latenten Vektoren kann verhindert werden, dass die Optimierung gewissermaßen in lokalen Minima stecken bleibt und stattdessen ein optimale Objektbewegungstrajektorie mit lediglich physikalisch möglichen oder realistischen Bewegungskonfigurationen resultiert. Dadurch kann die Zuverlässigkeit der Bildrekonstruktion deutlich erhöht werden.

Gemäß zumindest einer Ausführungsform des Verfahrens ist der trainierte Algorithmus dazu ausgelegt, einem latenten Vektor des latenten Raums, insbesondere jedem beliebigen latenten Vektor des latenten Raums, als Objektbewegungstrajektorie eine Zeitreihe von Rotationen und Translationen im dreidimensionalen Raum zuzuweisen.

Jede Rotation kann insbesondere durch drei Rotationsparameter, insbesondere drei Rotationswinkel, definiert werden und jede Translation durch drei Translationswerte, also Verschiebungen in drei verschiedene räumliche Richtungen. Die Zeitreihe von Rotationen und Translationen kann eine sechsdimensionale Zeitreihe für die drei Rotations- und drei Translationsparameter sein oder kann auch sechs einzelne Zeitreihen für die einzelnen Freiheitsgrade beziehungsweise Rotations- und Translationsparameter beinhalten. Die Die Zeitreihe von Rotationen und Translationen kann auch eine Zeitreihe von Rotationsmatrizen und Translationsvektoren sein.

Insbesondere kann die Vielzahl von Projektionsbildern K Projektionsbilder enthalten, wobei jedes Projektionsbild einem von K aufeinanderfolgenden Aufnahmezeiträumen entspricht und die Zeitreihe K Rotationen und K Translationen enthält.

K ist dabei eine ganze Zahl größer als 1 und kann beispielsweise im Bereich 100 bis 1.000 liegen. Insbesondere für CBCT kann die Anzahl der Projektionsbilder in einem solchen Bereich liegen, beispielsweise im Bereich 300 bis 600.

Während jedes Aufnahmezeitraums wird also insbesondere eines der K Projektionsbilder erzeugt. Jedem Projektionsbild sind also eine Rotation und eine Translation in jeweils drei Dimensionen zugeordnet. Mit dieser Zuordnung kann wie oben beschrieben die geometrische Operation zur Bildrekonstruktion für das entsprechende Projektionsbild korrigiert werden, um die Bewegungskompensation zu realisieren.

Gemäß zumindest einer Ausführungsform wird zum Erzeugen des trainierten Algorithmus eine Vielzahl von Trainingsobjektbewegungstrajektorien erzeugt wird, welche jeweils eine tatsächliche Bewegung eines Testobjekts beschreiben. Für jede der Trainingsobjektbewegungstrajektorien wird ein Trainingsvektor in dem latenten Raum erzeugt, insbesondere durch Anwendung eines codierenden neuronalen Netzwerks auf die jeweilige Trainingsobjektbewegungstrajektorie. Ein rekonstruierendes neuronales Netzwerk wird auf den Trainingsvektor angewendet, um eine rekonstruierte Objektbewegungstrajektorie zu erzeugen.

Wenigstens eine vordefinierte Verlustfunktion wird basierend auf der rekonstruierten Objektbewegungstrajektorie ausgewertet, gegebenenfalls unter Verwendung eines oder mehrerer weiterer neuronaler Netzwerke. Netzwerkparameter, insbesondere Gewichtungsfaktoren und/oder Bias-Faktoren, desrekonstruierenden neuronalen Netzwerk, insbesondere des rekonstruierenden neuronalen Netzwerks und des codierenden neuronalen Netzwerks und, gegebenenfalls des einen oder der mehreren weiteren neuronalen Netzwerke, werden abhängig von einem Ergebnis der Auswertung der wenigstens einen Verlustfunktion angepasst.

Das codierende neuronale Netzwerk kann beispielsweise ein Encodermodul eines Autoencoders sein und das rekonstruierende neuronale Netzwerk ein Decodermodul des Autoencoders. Im Falle eines GAN kann das rekonstruierende neuronale Netzwerk ein Generatormodul des GAN sein und das encodierende neuronale Netzwerk ein zusätzlich zum GAN bereitgestelltes neuronales Netzwerk. Das wenigstens eine weitere neuronale Netzwerk kann dann ein Diskriminatormodul des GAN sein.

Gemäß zumindest einer Ausführungsform ist der trainierte Algorithmus als Generatormodul eines GAN-Netzwerks ausgestaltet.

GAN-Netzwerke haben sich als zuverlässige Werkzeuge herausgestellt, um komplexe Eingabedaten in latenten Räumen zu codieren und zuverlässige Rekonstruktionen der Eingabedaten zu ermöglichen.

Ein GAN-Netzwerk beinhaltet zunächst das Generatormodul wie auch ein entsprechendes Diskriminatormodul. Das Diskriminatormodul wird allerdings nach abgeschlossenem Training nicht weiter benötigt.

Gemäß zumindest einer Ausführungsform, bei der der trainierte Algorithmus als Generatormodul des GAN-Netzwerks ausgestaltet ist, wird zum Erzeugen des trainierten Algorithmus, also insbesondere zum Trainieren des Algorithmus, die Vielzahl von Trainingsobjektbewegungstrajektorien erzeugt, welche jeweils eine tatsächliche Bewegung eines Testobjekts beschreiben. Für jede der Trainingsobjektbewegungstrajektorien wird ein Trainingsvektor im latenten Raum erzeugt, indem ein als Encoder ausgestaltetes künstliches neuronales Netzwerk auf die jeweilige Trainingsobjektbewegungstrajektorie angewendet wird. Das Generatormodul und das Diskriminatormodul des GAN-Netzwerks werden unter Verwendung der Trainingsvektoren als Eingangsdaten trainiert.

Das Trainieren des GAN-Netzwerks, insbesondere des Generatormoduls und des Diskriminatormoduls, kann in bekannter Weise erfolgen. Dabei erzeugt das Generatormodul basierend auf dem jeweiligen Trainingsvektor eine rekonstruierte Objektbewegungstrajektorie und das Diskriminatormodul entscheidet, ob es sich bei der rekonstruierten Objektbewegungstrajektorie um eine solche rekonstruierte Objektbewegungstrajektorie handelt, auch als "fake sample" bezeichnet, oder eine echte Trainingsobjektbewegungstrajektorie, auch als "real sample" bezeichnet. Das Training wird so lange durchgeführt, bis das Diskriminatormodul echte Trainingsobjektbewegungstrajektorien nicht mehr von rekonstruierten Objektbewegungstrajektorien unterscheiden kann. Nunmehr kann das Generatormodul zur zuverlässigen Erzeugung von Objektbewegungstrajektorien auf der Grundlage beliebiger latenter Vektoren in dem latenten Raum verwendet werden.

Insbesondere können eine bekannte Generator-Verlustfunktion und eine bekannte Diskriminator-Verlustfunktion eingesetzt werden, um das Generatormodul und das Diskriminatormodul entsprechend zu trainieren. Das Encodermodul kann vortrainiert sein, also ein bekanntes codierendes Encodermodul sein oder kann vor dem Trainieren des GAN-Netzwerks oder zusammen mit dem GAN-Netzwerk trainiert werden.

Das Encodermodul, welches den jeweiligen Trainingsvektor erzeugt, ist im engeren Sinne nicht Teil des GAN-Netzwerks. Vielmehr können GAN-Netzwerke auf beliebigen Eingaben, insbesondere Rausch-Vektoren, trainiert werden. Im vorliegenden Fall wird jedoch gezielt basierend auf tatsächlichen Trainingsobjektbewegungstrajektorien, welche physikalisch realistische Bewegungskonstellationen darstellen, erzeugt, sodass umgekehrt auch die von dem Generatormodul erzeugten Objektbewegungstrajektorien physikalisch realistischen Bewegungskonstellationen entsprechen. Das Encodermodul wird ebenso wie das Diskriminatormodul nach vollendetem Training nicht weiter benötigt.

Gemäß zumindest einer Ausführungsform ist der trainierte Algorithmus als Decodermodul eines Autoencoders ausgestaltet.

Autoencoder bestehen im Wesentlichen aus einem Encodermodul, welches eine entsprechenden Eingabe, hier beispielsweise eine Trainingsobjektbewegungstrajektorie, in einen latenten Vektor überführen kann und einem Decodermodul, welches die Eingabe basierend auf dem latenten Vektor rekonstruieren kann. Das Encodermodul wird nach abgeschlossenem Training nicht weiter benötigt.

Autoencoder haben sich als zuverlässige Werkzeuge herausgestellt, um komplexe Eingabedaten in latenten Räumen zu codieren und zuverlässige Rekonstruktionen der Eingabedaten zu ermöglichen. Autoencoder haben darüber hinaus den Vorteil, dass die Regularisierung während des Trainings im Vergleich zu anderen Netzwerken unproblematisch ist.

Auch zum Erzeugen beziehungsweise Trainieren des Autoencoders kann die Vielzahl von Trainingsobjektbewegungstrajektorien erzeugt werden, welche jeweils eine tatsächliche Bewegung eines Testobjekts beschreiben. Für jede der Trainingsobjektbewegungstrajektorien wird ein Trainingsvektor in dem latenten Raum erzeugt, indem das Encodermodul des Autoencoders oder Variational Autoencoders auf die jeweilige Trainingsobjektbewegungstrajektorie angewendet wird. Mittels des Decodermoduls wird basierend auf dem Trainingsvektor eine rekonstruierte Objektbewegungstrajektorie erzeugt und eine Verlustfunktion wird basierend auf einer Abweichung der rekonstruierten **Ob**jektbewegungstrajektorie von der Trainingsobjektbewegungstrajektorie ausgewertet. Netzwerkparameter des Autoencoders, insbesondere des Encodermoduls und des Decodermoduls, werden abhängig von einem Ergebnis der Auswertung der Verlustfunktion angepasst.

Zum Anpassen der Netzwerkparameter können bekannte Verfahren und bekannte Verlustfunktionen eingesetzt werden.

Das Encodermodul wird vorliegend gezielt basierend auf tatsächlichen Trainingsobjektbewegungstrajektorien trainiert, welche physikalisch realistische Bewegungskonstellationen darstellen, um die Trainingsvektoren zu erzeugen, sodass umgekehrt auch die von dem Decodermodul erzeugten Objektbewegungstrajektorien physikalisch realistischen Bewegungskonstellationen entsprechen.

Gemäß zumindest einer Ausführungsform wird für jede der Trainingsobjektbewegungstrajektorien zum Erzeugen der jeweiligen Trainingsobjektbewegungstrajektorie das Testobjekt während eines entsprechenden Testzeitraums mittels wenigstens einer Kamera überwacht und die jeweilige Trainingsobjektbewegungstrajektorie wird basierend auf während der Überwachung mittels der wenigstens einen Kamera erzeugter Kamerabilder bestimmt.

Insbesondere kann eine Position und/oder Orientierung des Testobjekts in einem Referenzkoordinatensystem basierend auf den Kamerabildern bestimmt werden und eine entsprechende Sequenz von Rotationen und Translationen beziehungsweise entsprechender Rotations- und Translationsparameter kann berechnet werden.

Gemäß zumindest einer Ausführungsform wird an dem Testobjekt wenigstens ein visueller Marker angebracht und die jeweilige Trainingsobjektbewegungstrajektorie wird basierend auf den entsprechenden Positionen des wenigstens einen visuellen Markers in den Kamerabildern bestimmt.

Insbesondere können aus den Positionen in den Kamerabildern die Position und/oder Orientierung im Referenzkoordinatensystem bestimmt werden, da die Positionen und Orientierungen der wenigstens einen Kamera in dem Referenzkoordinatensystem bekannt sind.

Auf diese Weise lässt sich eine zuverlässige Aufzeichnung ausschließlich physikalisch realistische Bewegungskonstellationen erreichen und dementsprechend die Zuverlässigkeit des trainierten Algorithmus und dementsprechend des gesamten erfindungsgemäßen Verfahrens erhöhen.

Bei dem Objekt kann es sich beispielsweise um einen Kopf eines Patienten handeln. Bei der röntgenbasierten Bildgebung des Kopfes lässt sich die beschriebene Bewegungskompensation mit besonders hoher Genauigkeit durchführen, da die Annahme starrer Bewegungen, insbesondere ohne Deformation des Objekts, besonders gut erfüllt ist.

Gemäß einem weiteren Aspekt der Erfindung wird auch ein Verfahren zur röntgenbasierten Bildgebung angegeben. Dabei wird eine Vielzahl von Projektionsbildern eines Objekts erzeugt und es wird ein Verfahren zur Erzeugung einer bewegungskompensierten Bildrekonstruktion gemäß der Erfindung basierend auf der Vielzahl von Projektionsbildern durchgeführt.

Insbesondere ist das Verfahren zur röntgenbasierten Bildgebung ein Kegelstrahl-Computertomographieverfahren.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Gemäß einem weiteren Aspekt der Erfindung wird eine Datenverarbeitungsvorrichtung angegeben, die wenigstens eine Recheneinheit aufweist. Die wenigstens eine Recheneinheit ist dazu eingerichtet oder angepasst, ein erfindungsgemäßes Verfahren zur Erzeugung einer bewegungskompensierten Bildrekonstruktion durchzuführen.

Gemäß einem weiteren Aspekt der Erfindung wird eine Vorrichtung zur röntgenbasierten Bildgebung angegeben. Die Vorrichtung weist eine Röntgenbildgebungsmodalität auf, die dazu eingerichtet ist, eine Vielzahl von Projektionsbildern eines Objekts zu erzeugen. Die Vorrichtung weist ein Speichergerät auf, das einen trainierten Algorithmus speichert, der dazu trainiert ist, einem latenten Vektor eines vorgegebenen latenten Raums eine Objektbewegungstrajektorie zuzuweisen. Die Vorrichtung weist wenigstens eine Recheneinheit auf, die dazu eingerichtet ist, eine optimale Objektbewegungstrajektorie durch Anwenden des trainierten Algorithmus auf einen optimalen latenten Vektor zu bestimmen und die bewegungskompensierte Bildrekonstruktion abhängig von der Vielzahl von Projektionsbildern unter der Annahme zu erzeugen, das Objekt habe sich während der Durchführung des Bildgebungsverfahrens gemäß der optimalen Objektbewegungstrajektorie bewegt.

Die wenigstens eine Recheneinheit ist dazu eingerichtet, eine vorgegebene Kostenfunktion unter Verwendung des latenten Vektors als Optimierungsparameter zu minimieren, um den optimalen latenten Vektor zu bestimmen und dabei für jeden Optimierungsschritt mit einem gegenwärtigen latenten Vektor durch Anwendung des trainierten Algorithmus auf den gegenwärtigen latenten Vektor eine gegenwärtige Objektbewegungstrajektorie zu bestimmen, und einen gegenwärtigen Wert der Kostenfunktion abhängig von der Vielzahl von Projektionsbildern unter der Annahme zu berechnen, das Objekt habe sich während der Durchführung des Bildgebungsverfahrens gemäß der gegenwärtigen Objektbewegungstrajektorie bewegt.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Weitere Ausführungsformen der erfindungsgemäßen Vorrichtung folgen unmittelbar aus den verschiedenen Ausgestaltungen der erfindungsgemäßen Verfahren und umgekehrt. Insbesondere lassen sich einzelne Merkmale und entsprechende Erläuterungen sowie Vorteile bezüglich der verschiedenen Ausführungsformen zu den erfindungsgemäßen Verfahren analog auf entsprechende Ausführungsformen der erfindungsgemäßen Vorrichtung übertragen. Insbesondere ist die erfindungsgemäße Vorrichtung zum Durchführen eines erfindungsgemäßen Verfahrens ausgebildet oder programmiert. Insbesondere führt die erfindungsgemäße Vorrichtung ein erfindungsgemäßes Verfahren durch.

Gemäß einem weiteren Aspekt der Erfindung wird ein erstes Computerprogramm mit ersten Befehlen angegeben. Bei Ausführung der ersten Befehle durch eine Datenverarbeitungsvorrichtung, insbesondere eine erfindungsgemäße Datenverarbeitungsvorrichtung, veranlassen die erste Befehle die Datenverarbeitungsvorrichtung dazu, ein erfindungsgemäßes Verfahren zur Erzeugung einer bewegungskompensierten Bildrekonstruktion durchzuführen.

Gemäß einem weiteren Aspekt der Erfindung wird ein zweites Computerprogramm mit zweiten Befehlen angegeben. Bei Ausführung der zweiten Befehle durch eine erfindungsgemäße Vorrichtung zur röntgenbasierten Bildgebung, insbesondere durch die wenigstens eine Recheneinheit der Vorrichtung, veranlassen die zweiten Befehle die Vorrichtung dazu, ein erfindungsgemäßes Verfahren zur Erzeugung einer bewegungskompensierten Bildrekonstruktion und/oder ein erfindungsgemäßes Verfahren zur röntgenbasierten Bildgebung durchzuführen.

Gemäß einem weiteren Aspekt der Erfindung wird ein computerlesbares Speichermedium angegeben, welches ein erstes Computerprogramm und/oder ein zweites Computerprogramm gemäß der Erfindung speichert.

Das erste Computerprogramm, das zweite Computerprogramm sowie das computerlesbare Speichermedium können als jeweilige Computerprogrammprodukte aufweisend die ersten und/oder die zweiten Befehle aufgefasst werden.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen:

FIG 1    eine schematische Darstellung einer beispielhaften Ausführungsform einer erfindungsgemäßen Vorrichtung zur röntgenbasierten Bildgebung,

FIG 2    eine schematische Darstellung verschiedener Parameterräume zur Parametrisierung von Objektbewegungstrajektorien,

FIG 3    eine schematische Darstellung einer Trainingsphase zum Trainieren eines Algorithmus zur Durchführung einer beispielhaften Ausführungsform eines erfindungsgemäßen Verfahrens zur Erzeugung einer bewegungskompensierten Bildrekonstruktion, und

FIG 4    ein schematisches Ablaufdiagramm einer weiteren beispielhaften Ausführungsform eines erfindungsgemäßen Verfahrens zur Erzeugung einer bewegungskompensierten Bildrekonstruktion.

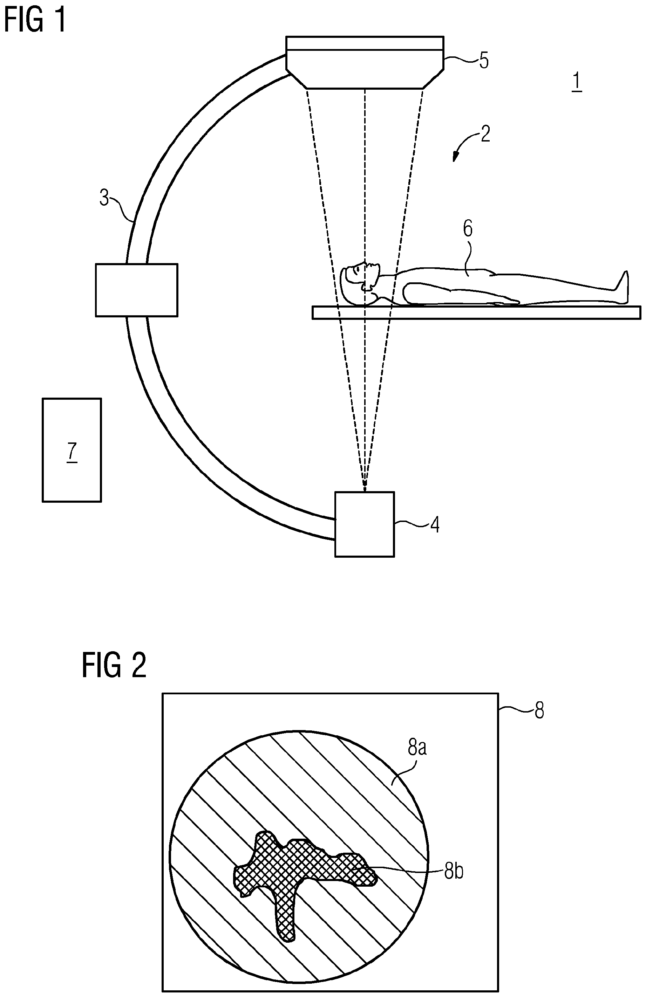

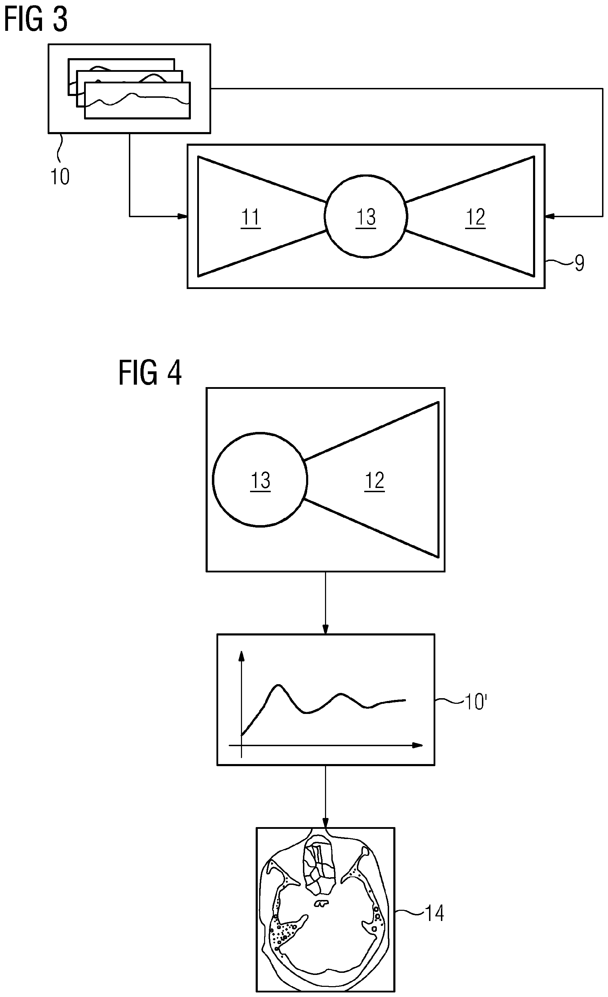

In FIG 1 ist schematisch eine erfindungsgemäße Vorrichtung 1 zur röntgenbasierten Bildgebung aufweisend eine Röntgenbildgebungsmodalität 2 dargestellt. Die Röntgenbildgebungsmodalität 2 ist beispielhaft als Gerät zur Kegelstrahl-Computertomographie, CBCT, dargestellt und weist beispielsweise einen C-Bogen 3 mit einer Röntgenquelle 4 und einem gegenüberliegend angeordneten Röntgendetektor 5 auf. Zwischen Röntgenquelle 4 und Röntgendetektor 5 ist ein Objekt 6, beispielsweise der Kopf eines Patienten, angeordnet. Des Weiteren weist die Vorrichtung 1 eine Recheneinheit 7 auf. Die Vorrichtung 1, insbesondere die Recheneinheit 7, weist ein Speichergerät auf (nicht dargestellt), das einen trainierten Algorithmus 12 (siehe FIG 3, FIG 4) speichert, der dazu trainiert ist, einem latenten Vektor eines vorgegebenen latenten Raums (siehe FIG 3, FIG 4) eine Objektbewegungstrajektorie zuzuweisen.

Die Vorrichtung 1 ist insbesondere dazu eingerichtet, ein erfindungsgemäßes Verfahren zur röntgenbasierten Bildgebung durchzuführen. Dazu erzeugt die Röntgenbildgebungsmodalität 2 eine Vielzahl von Projektionsbildern des Objekts 6, insbesondere gemäß einem bekannten Protokoll zur CBCT. Beispielsweise können etwa 500 Projektionsbilder des Objekts 6 erzeugt werden. Des Weiteren wird ein erfindungsgemäßes Verfahren zur Erzeugung einer bewegungskompensierten Bildrekonstruktion 14 durchgeführt. Ein schematisches Ablaufdiagramm eines solchen Verfahrens ist in FIG 4 dargestellt.

Insbesondere wird dabei eine optimale Objektbewegungstrajektorie 10' für das Objekt 6 durch Anwenden des trainierten Algorithmus 12 auf einen optimalen latenten Vektor in dem latenten Raum 13 erzeugt und die bewegungskompensierte Bildrekonstruktion 14 wird abhängig von der Vielzahl von Projektionsbildern unter der Annahme erzeugt, das Objekt 6 habe sich während der Durchführung des Bildgebungsverfahrens, also während der Erzeugung der Projektionsbilder, gemäß der optimalen Objektbewegungstrajektorie 10' bewegt.

Der optimale latente Vektor wird dabei bestimmt, indem eine vorgegebene Kostenfunktion unter Verwendung des latenten Vektors als Optimierungsparameter minimiert wird. Für jeden Optimierungsschritt mit einem gegenwärtigen latenten Vektor wird durch Anwendung des trainierten Algorithmus 12 auf den gegenwärtigen latenten Vektor eine gegenwärtige Objektbewegungstrajektorie bestimmt. Eine gegenwärtige Bildrekonstruktion wird abhängig von der Vielzahl von Projektionsbildern erzeugt. Dies erfolgt unter der Annahme, das Objekt 6 habe sich während der Durchführung des Bildgebungsverfahrens gemäß der gegenwärtigen Objektbewegungstrajektorie bewegt. Ein gegenwärtiger Wert der Kostenfunktion wird abhängig von der gegenwärtigen Bildrekonstruktion berechnet.

Alternativ kann der gegenwärtige Wert der Kostenfunktion abhängig von einem vorgegebenen Satz von Konsistenzbedingungen betreffend die Vielzahl von Projektionsbildern berechnet werden, sodass bei den einzelnen Optimierungsschritten keine gegenwärtige Bildrekonstruktion erzeugt werden muss.

Bei dem trainierten Algorithmus 12 kann es sich beispielsweise um ein Decodermodul 12 eines Autoencoders 9 handeln, wie schematisch in FIG 3 dargestellt. Der Autoencoder 9 weist während der Trainingsphase ein Encodermodul 11 auf, das eine Trainingsobjektbewegungstrajektorie 10 in einen entsprechenden latenten Vektor des latenten Raums 13 codieren kann. Das Decodermodul 12 rekonstruiert die Trainingsobjektbewegungstrajektorie 10 basierend auf dem jeweiligen latenten Vektor.

Das Modell einer starren Bewegung ohne jegliche Einschränkung basiert auf Rotationen und Translation, typischerweise definiert als die spezielle euklidische Gruppe in drei Dimensionen SE(3). Dieses Bewegungsmodell ist sehr leistungsfähig, allerdings sind die meisten Bewegungskonstellationen, die durch SE(3) beschrieben werden können, nicht realistisch. Die Bewegungen, die prinzipiell durch Rotationen und Translation beschrieben werden können, ist in FIG 2 schematisch als Quadrat 8 dargestellt. Insbesondere ist in FIG 2 eine Mannigfaltigkeit der möglichen Bewegungen als das Quadrat 8 schematisch dargestellt.

Die Abtastung der Bewegung über Spline-Knoten reduziert bereits die Menge der möglichen unrealistischen Bewegungskonstellationen. Die Bewegungen, die prinzipiell durch Splines beschrieben werden können, ist in FIG 2 schematisch als Kreis 8a dargestellt, insbesondere als Submannigfaltigkeit der Mannigfaltigkeit aller möglichen Bewegungen. Dennoch sind immer noch viele unrealistische Bewegungskonstellationen möglich. Bei Patienten, die auf einem Tisch liegen und durch die menschliche Anatomie eingeschränkt sind, ist nur eine Teilmenge von SE(3) physikalisch möglich. Dieser Anteil ist in Fig. 2 schematisch unregelmäßiger Bereich 8b dargestellt und repräsentiert die Teilmenge von SE(3), die nur physikalisch beziehungsweise anatomisch mögliche Bewegungskonstellationen enthält, insbesondere als kleinere Submannigfaltigkeit der durch Splines beschreibbaren Bewegungen.

In verschiedenen Ausführungsformen der Erfindung werden in einem ersten Schritt Bewegungstrajektorien von echten Patienten als Testobjektbewegungstrajektorien 10 abgetastet. Dies kann zum Beispiel durch Tracking geschehen. Ein Patient trägt zum Beispiel Marker auf dem Kopf, und ein Kamerasystem verfolgt diese Marker. Anhand der Marker und des Kamerasystems kann eine Bewegungstrajektorie des Patienten berechnet werden. Durch die Aufzeichnung beispielsweise mehrerer Patienten über mehrere Stunden entsteht eine sehr große Datenbank mit realistischen Testobjektbewegungstrajektorien 10.

Basierend auf einem Satz realistischer Testobjektbewegungstrajektorien 10 kann der Autoencoder 9 zur Kodierung und Dekodierung dieser Testobjektbewegungstrajektorien 10 verwendet werden. Der Autoencoder 9 kann als regulärer Autoencoder oder als Variational Autoencoder 9 ausgestaltet sein. Ein Autoencoder 9 ist insbesondere dadurch definiert, dass er die Eingabe des Netzwerk in der Ausgabeschicht reproduziert. Durch die Einführung eines Engpasses in der Mitte des Netzwerks, also nach dem Encodermodul 11, ist der Autoencoder 9 gezwungen, den latenten Raum 13 der Testobjektbewegungstrajektorien 10 zu erlernen, anstatt eine einfachen Identitätsabbildung durchzuführen.

Das Encodermodul 11 kann ein implizites neuronales Netz sein, zum Beispiel gemäß einer SIREN-Architektur, wie in der Publikation V. Sitzmann, J. Martel, A. Bergman, A. Lindell, G. Wetzstein: "Implicit neural representations with periodic activation functions", Advances in Neural Information Processing Systems. 2020;33:7462-73 beschrieben. Ein Vorteil der SIREN-Architektur ist die Fähigkeit, Ableitungen höherer Ordnung zu berechnen. Dies ist möglich, weil die klassische Re-Lu-Aktivierungsfunktion durch eine Sinusfunktion ersetzt ist. Infolgedessen sind eine Jacobische Regularisierung oder eine Bending-Regularisierung möglich, die eine glatte und realistische Abbildung vom latenten Raum auf den Ausgaberaum erzwingt, sodass zum Beispiel kleine Änderungen im latenten Raum 13 zu kleinen Änderungen im Ausgaberaum führen. Man beachte, dass diese Art von Verhalten auch durch den Variations-Autoencoder 9 erzwungen wird. Diese Eigenschaft kann wichtig sein, wenn der latente Raum 13 als Parametrisierung für die Bewegungskompensation verwendet wird.

Nach erfolgreichem Training kann das Decodermodul 12 des Autoencoders 9 extrahiert werden und bei der Erzeugung der Objektbewegungstrajektorien verwendet werden.

Ein GAN kann als Alternative zu dem Autoencoder 9 verwendet werden. In diesem Fall wäre die Eingabe für das GAN wiederum ein latenter Vektor, das Generatormodul des GAN würde einen latenten Vektor auf eine Objektbewegungstrajektorie abbilden, und der Verlust würde auf der Unterscheidung zwischen echten und sogenannten falschen Objektbewegungstrajektorien beruhen.

Nach dem Training kann das Decodermodul 12 auf der Grundlage eines gegenwärtigen latenten Vektors eine gegenwärtige Objektbewegungstrajektorie erzeugen. Diese Objektbewegungstrajektorie kann dann in Projektionsmatrizen übersetzt und im Rekonstruktionsalgorithmus verwendet werden, um eine gegenwärtige Bildrekonstruktion zu erstellen, die auf der gegenwärtigen Objektbewegungstrajektorie basiert. Im Vergleich zur Parametrisierung mit Splines resultieren jedoch nur Bewegungskonstellationen, die physikalisch sinnvoll sind.

Der latente Raum 13 stellt nun die Parametrisierung für die Objektbewegungstrajektorie dar. Das heißt, die Parametrisierung X ist der latente Vektor. Die Bewegungskompensation kann dann in der regulären Notation durch

$$X' = argmin_X c\big(r(I, X)\big),$$

definiert werden beziehungsweise durch

$$X' = argmin_X c(I, X),$$

falls die Kostenfunktion c() auf Konsistenzbedingungen, insbesondere epipolaren Konsistenzbedingungen, basiert. Die Lösung dieses Minimierungsproblems ergibt den optimalen latenten Vektor X', der die Bewegungskompensation löst.

**Patentansprüche**

1. Verfahren zur Erzeugung einer bewegungskompensierten Bildrekonstruktion (14) bei einem röntgenbasierten Bildgebungsverfahren, wobei

   i) eine Vielzahl während der Durchführung des Bildgebungsverfahrens erzeugter Projektionsbilder eines Objekts (6) bereitgestellt wird;
   ii) ein trainierter Algorithmus bereitgestellt wird, der dazu trainiert ist, einem latenten Vektor eines vorgegebenen latenten Raums (13) eine Objektbewegungstrajektorie zuzuweisen;
   iii) eine optimale Objektbewegungstrajektorie (10') durch Anwendung des trainierten Algorithmus (12) auf einen optimalen latenten Vektor bestimmt wird und die bewegungskompensierte Bildrekonstruktion (14) abhängig von der Vielzahl von Projektionsbildern unter der Annahme erzeugt wird, das Objekt (6) habe sich während der Durchführung des Bildgebungsverfahrens gemäß der optimalen Objektbewegungstrajektorie (10') bewegt; und
   iv) der optimale latente Vektor bestimmt wird, indem eine vorgegebene Kostenfunktion unter Verwendung des latenten Vektors als Optimierungsparameter minimiert wird,

   wobei für jeden Optimierungsschritt mit einem gegenwärtigen latenten Vektor

   - durch Anwendung des trainierten Algorithmus (12) auf den gegenwärtigen latenten Vektor eine gegenwärtige Objektbewegungstrajektorie bestimmt wird; und
   - ein gegenwärtiger Wert der Kostenfunktion abhängig von der Vielzahl von Projektionsbildern unter der Annahme berechnet wird, das Objekt (6) habe sich während der Durchführung des Bildgebungsverfahrens gemäß der gegenwärtigen Objektbewegungstrajektorie bewegt.

2. Verfahren nach Anspruch 1, wobei der trainierte Algorithmus (12) dazu ausgelegt ist, einem latenten Vektor des latenten Raums (13) als Objektbewegungstrajektorie eine Zeitreihe von Rotationen und Translationen im dreidimensionalen Raum zuzuweisen.

3. Verfahren nach Anspruch 2, wobei

    - die Vielzahl von Projektionsbildern K Projektionsbilder enthält, wobei jedes Projektionsbild einem von K aufeinanderfolgenden Aufnahmezeiträumen entspricht; und
    - die Zeitreihe K Rotationen und K Translationen enthält.

4. Verfahren nach einem der vorhergehenden Ansprüche, wobei der trainierte Algorithmus (12) als Decodermodul (12) eines Autoencoders (9) oder eines Variational Autoencoders (9) ausgestaltet ist.

5. Verfahren Anspruch 4, wobei zum Erzeugen des trainierten Algorithmus (12) eine Vielzahl von Trainingsobjektbewegungstrajektorien (10) erzeugt wird, welche jeweils eine tatsächliche Bewegung eines Testobjekts beschreiben, und für jede der Trainingsobjektbewegungstrajektorien (10)

    - ein Trainingsvektor in dem latenten Raum (13) erzeugt wird, indem ein Encodermodul (11) des Autoencoders (9) oder Variational Autoencoders (9) auf die jeweilige Trainingsobjektbewegungstrajektorie (10) angewendet wird;
    - mittels des Decodermoduls (12) basierend auf dem Trainingsvektor eine rekonstruierte Objektbewegungstrajektorie erzeugt wird;
    - eine Verlustfunktion basierend auf einer Abweichung der rekonstruierten Objektbewegungstrajektorie von der Trainingsobjektbewegungstrajektorie (10) ausgewertet wird; und
    - Netzwerkparameter des Autoencoders (9) oder Variational Autoencoders (9) abhängig von einem Ergebnis der Auswertung der Verlustfunktion angepasst werden.

6. Verfahren nach einem der Ansprüche 1 bis 4, wobei der trainierte Algorithmus (12) als Generatormodul eines GAN-Netzwerks ausgestaltet ist.

7. Verfahren nach Anspruch 6, wobei

    - zum Erzeugen des trainierten Algorithmus (12) eine Vielzahl von Trainingsobjektbewegungstrajektorien (10) erzeugt wird, welche jeweils eine tatsächliche Bewegung eines Testobjekts beschreiben;

    - für jede der Trainingsobjektbewegungstrajektorien (10) ein Trainingsvektor in dem latenten Raum (13) erzeugt wird, indem ein als Encoder ausgestaltetes künstliches neuronales Netzwerk auf die jeweilige Trainingsobjektbewegungstrajektorie (10) angewendet wird;
    - das Generatormodul und ein Diskriminatormodul des GAN-Netzwerks unter Verwendung der Trainingsvektoren als Eingangsdaten trainiert werden.

8. Verfahren nach einem der Ansprüche 5 oder 7, wobei für jede der Trainingsobjektbewegungstrajektorien (10) zum Erzeugen der jeweiligen Trainingsobjektbewegungstrajektorie (10) das Testobjekt während eines entsprechenden Testzeitraums mittels wenigstens einer Kamera überwacht wird und die jeweilige Trainingsobjektbewegungstrajektorie (10) basierend auf während der Überwachung mittels der wenigstens einen Kamera erzeugter Kamerabilder bestimmt wird.

9. Verfahren nach Anspruch 8, wobei an dem Testobjekt (6) wenigstens ein visueller Marker angebracht wird und die jeweilige Trainingsobjektbewegungstrajektorie (10) basierend auf entsprechenden Positionen des wenigstens einen visuellen Markers in den Kamerabildern bestimmt wird.

10. Verfahren nach einem der vorhergehenden Ansprüche, wobei für jeden der Optimierungsschritte

    - eine gegenwärtige Bildrekonstruktion abhängig von der Vielzahl von Projektionsbildern unter der Annahme erzeugt wird, das Objekt (6) habe sich während der Durchführung des Bildgebungsverfahrens gemäß der gegenwärtigen Objektbewegungstrajektorie bewegt, und der gegenwärtige Wert der Kostenfunktion abhängig von der gegenwärtigen Bildrekonstruktion berechnet wird; oder
    - der gegenwärtige Wert der Kostenfunktion abhängig von einem vorgegebenen Satz von Konsistenzbedingungen betreffend die Vielzahl von Projektionsbildern berechnet wird.

11. Verfahren zur röntgenbasierten Bildgebung, wobei eine Vielzahl von Projektionsbildern eines Objekts (6) erzeugt wird und ein Verfahren zur Erzeugung einer bewegungskompensierten Bildrekonstruktion (14) gemäß einem der vorhergehenden Ansprüche basierend auf der Vielzahl von Projektionsbildern durchgeführt wird.

12. Verfahren nach Anspruch 11, wobei das Verfahren ein Kegelstrahl-Computertomographieverfahren ist.

13. Datenverarbeitungsvorrichtung (7) mit wenigstens einer Recheneinheit (7), die dazu angepasst ist, ein Verfahren zur Erzeugung einer bewegungskompensierten Bildrekonstruktion (14) gemäß einem der vorhergehenden Ansprüche 1 bis 10 durchzuführen.

14. Vorrichtung (1) zur röntgenbasierten Bildgebung aufweisend eine Röntgenbildgebungsmodalität (2), die dazu eingerichtet ist, eine Vielzahl von Projektionsbildern eines Objekts (6) zu erzeugen, ein Speichergerät, das einen trainierten Algorithmus (12) speichert, der dazu trainiert ist, einem latenten Vektor eines vorgegebenen latenten Raums (13) eine Objektbewegungstrajektorie zuzuweisen, sowie wenigstens eine Recheneinheit (7), die dazu eingerichtet ist,

- eine optimale Objektbewegungstrajektorie (10') durch Anwendung des trainierten Algorithmus (12) auf einen optimalen latenten Vektor zu bestimmen und die bewegungskompensierte Bildrekonstruktion (14) abhängig von der Vielzahl von Projektionsbildern unter der Annahme zu erzeugen, das Objekt (6) habe sich während der Durchführung des Bildgebungsverfahrens gemäß der optimalen Objektbewegungstrajektorie (10') bewegt; und
- eine vorgegebene Kostenfunktion unter Verwendung des latenten Vektors als Optimierungsparameter zu minimieren, um den optimalen latenten Vektor zu bestimmen und dabei für jeden Optimierungsschritt mit einem gegenwärtigen latenten Vektor durch Anwendung des trainierten Algorithmus (12) auf den gegenwärtigen latenten Vektor eine gegenwärtige Objektbewegungstrajektorie zu bestimmen, und einen gegenwärtigen Wert der Kostenfunktion abhängig von der Vielzahl von Projektionsbildern unter der Annahme zu berechnen, das Objekt (6) habe sich während der Durchführung des Bildgebungsverfahrens gemäß der gegenwärtigen Objektbewegungstrajektorie bewegt.

15. Computerprogrammprodukt aufweisend

- erste Befehle, die bei Ausführung durch eine Datenverarbeitungsvorrichtung (7) die Datenverarbeitungsvorrichtung (7) dazu veranlassen, ein Verfahren gemäß einem der Ansprüche 1 bis 10 auszuführen; und/oder
- zweite Befehle, die bei Ausführung durch eine Vorrichtung (1) nach Anspruch 14 die Vorrichtung (1) dazu veranlassen, ein Verfahren gemäß einem der Ansprüche 1 bis 12 durchzuführen.

**Claims**

1. Method for creation of a movement-compensated image reconstruction (14) for an x-ray based imaging method, wherein

i) a plurality of projection images of an object (6), created when the imaging method is being carried out, is provided;
ii) a trained algorithm is provided, which is trained to allocate an object movement trajectory to a latent vector of a predetermined latent space (13);
iii) an optimal object movement trajectory (10') is determined by application of the trained algorithm (12) to an optimal latent vector and the movement-compensated image reconstruction (14) is created depending on the plurality of projection images, with the assumption that the object (6) would have moved in accordance with the optimal object movement trajectory (10') while the imaging method was being carried out; and
iv) the optimal latent vector is determined by a predetermined cost function being minimised using the latent vector as the optimisation parameter,

wherein, for each optimisation step with a current latent vector

- a current object movement trajectory is determined by application of the trained algorithm (12) to the current latent vector; and
- a current value of the cost function is computed depending on the plurality of projection images, with the assumption that the object (6) would have moved in accordance with the current object movement trajectory while the imaging method was being carried out.

2. Method according to claim 1, wherein the trained algorithm (12) is designed to allocate a time series of rotations and translations in three-dimensional space to a latent vector of the latent space (13) as an object movement trajectory.

3. Method according to claim 2, wherein

- the plurality of projection images contains K projection images, wherein each projection image corresponds to one of K consecutive recording time spaces; and
- the time series contains K rotations and K translations.

4. Method according to one of the preceding claims, wherein the trained algorithm (12) is embodied as a decoder module (12) of an autoencoder (9) or of a variational autoencoder (9).

5. Method according to claim 4 wherein, for creation of the trained algorithm (12), a plurality of training object movement trajectories (10) is created, which each describe an actual movement of a test object, and for each of the training object movement trajectories (10)

- a training vector in the latent space (13) is created, by an encoder module (11) of the autoencoder (9) or the variational autoencoder (9) being applied to the respective training object movement trajectory (10);
- a reconstructed object movement trajectory is created by means of the decoder module (12) based on the training vector;
- a loss function based on a deviation of the reconstructed object movement trajectory from the training object movement trajectory (10) is evaluated; and
- network parameters of the autoencoder (9) or variational autoencoder (9) are adapted depending on a result of the evaluation of the loss function.

6. Method according to one of claims 1 to 4, wherein the trained algorithm (12) is embodied as a generator module of a GAN network.

7. Method according to claim 6, wherein

- for creation of the trained algorithm (12), a plurality of training object movement trajectories (10) is created, which each describe an actual movement of a test object;
- for each of the training object movement trajectories (10) a training vector in the latent space (13) is created, by an artificial neural network embodied as an encoder being applied to the respective training object movement trajectory (10);
- the generator module and a discriminator module of the GAN network are trained using the training vectors as input data.

8. Method according to one of claims 5 or 7, wherein for each of the training object movement trajectories (10), for creation of the respective training object movement trajectory (10), the test object is monitored during a corresponding test period by means of at least one camera and the respective training object movement trajectory (10) is determined based on camera images created by means of the at least one camera during the monitoring.

9. Method according to claim 8, wherein at least one visual marker is attached to the test object (6) and the respective training object movement trajectory (10) is determined based on corresponding positions of the at least one visual marker in the camera images.

10. Method according to one of the preceding claims, wherein, for each of the optimisation steps

- a current image reconstruction is created depending on the plurality of projection images, with the assumption that the object (6) would have moved in accordance with the current object movement trajectory while the imaging method was being carried out, and the current value of the cost function is computed depending on the current image reconstruction; or
- the current value of the cost function is computed depending on a predetermined set of consistency conditions relating to the plurality of projection images.

11. Method for x-ray based imaging, wherein a plurality of projection images of an object (6) is created and a method for creation of a movement-compensated image reconstruction (14) in accordance with one of the preceding claims is carried out based on the plurality of projection images.

12. Method according to claim 11, wherein the method is a cone-beam computed tomography method.

13. Data processing facility (7) with at least one processing unit (7), which is adapted to carry out a method for creation of a movement-compensated image reconstruction (14) according to one of the preceding claims 1 to 10.

14. Apparatus (1) for x-ray based imaging having an x-ray imaging modality (2), which is configured to create a plurality of projection images of an object (6), a memory device, which stores a trained algorithm (12), which is trained to allocate an object movement trajectory to a latent vector of a predetermined latent space (13), as well as at least one processing unit (7), which is configured,

- to determine an optimal object movement trajectory (10') by application of the trained algorithm (12) to an optimal latent vector and to create the movement-compensated image reconstruction (14) depending on the plurality of projection images, with the assumption that the object (6) would have moved in accordance with the optimal object movement trajectory (10') while the imaging method was being carried out; and
- to minimise a predetermined cost function using the latent vector as an optimisation parameter, in order to determine the optimal latent vector and, in doing so, to determine a current object movement trajectory for each optimisation step with a current latent vector by application of the trained algorithm (12) to the current latent vector, and to compute a current value of the cost function depending on the plurality of projection images, with the assumption that the object (6) would have moved in accordance with the current object movement trajectory while the imaging method was being carried out.

15. Computer program product having

- first instructions that, when executed by a data processing facility (7), cause said data processing facility (7) to carry out a method according to one of claims 1 to 10; and/or
- second instructions that, when executed by an apparatus (1) according to claim 14, cause said apparatus (1) to carry out a method according to one of claims 1 to 12.

## Revendications

1. Procédé de production d'une reconstruction (14) d'image à compensation de mouvement dans un procédé d'imagerie à base de rayons X**,** dans lequel

i) on se procure une pluralité d'images de projection d'un objet (6) produites pendant l'exécution du procédé d'imagerie ;
ii) on se procure un algorithme entraîné, qui est entraîné pour affecter une trajectoire de mouvement d'objet à un vecteur latent d'un espace (13) latent donné à l'avance ;
iii) on détermine une trajectoire (10') de mouvement d'objet optimale en appliquant l'algorithme (12) entraîné à un vecteur latent optimum et on produit la reconstruction (14) d'image à compensation de mouvement en fonction de la pluralité d'images de projection en supposant que l'objet (6) s'est, pendant l'exécution du procédé d'imagerie, mis en mouvement suivant la trajectoire (10') de mouvement d'objet optimale ; et
iv) on détermine le vecteur latent optimum en minimisant, en utilisant le vecteur latent comme paramètre d'optimisation, une fonction de coût donnée à l'avance,

dans lequel, pour chaque stade d'optimisation avec un vecteur latent présent

- en appliquant l'algorithme (12) entraîné au vecteur latent présent, on détermine une trajectoire de mouvement d'objet présente ; et

- on calcule une valeur présente de la fonction de coût en fonction de la pluralité d'images de projection en supposant que l'objet (6) s'est, pendant l'exécution du procédé d'imagerie, mis en mouvement suivant la trajectoire de mouvement d'objet présente.

2. Procédé suivant la revendication 1, dans lequel l'algorithme (12) entraîné est conçu pour affecter à un vecteur latent de l'espace (13) latent comme trajectoire de mouvement d'objet une série temporelle de rotations et de translations dans l'espace en trois dimensions.

3. Procédé suivant la revendication 2, dans lequel

- la pluralité d'images de projection contient K images de projection, dans lequel chaque image de projection correspond à l'un de K laps de temps d'enregistrement successifs ; et
- la série temporelle contient K rotations et K translations.

4. Procédé suivant l'une des revendications précédentes, dans lequel l'algorithme (12) entraîné est conformé en module (12) de décodeur d'un autocodeur (9) ou d'un autocodeur (9) variationnel.

5. Procédé suivant la revendication 4, dans lequel pour la production de l'algorithme (12) entraîné, on produit une pluralité de trajectoires (10) de mouvement d'objet d'entraînement, qui décrivent chacune un mouvement réel d'un objet de test et pour chaque trajectoire (10) de mouvement d'objet d'entraînement

- on produit un vecteur d'entraînement dans l'espace (13) latent en appliquant un module (11) de codeur de l'autocodeur (9) ou de l'autocodeur (9) variationnel à la trajectoire (10) respective de mouvement d'objet d'entraînement ;
- au moyen du module (12) de décodeur on produit sur la base du vecteur d'entraînement une trajectoire de mouvement d'objet reconstruite ;
- on évalue une fonction de perte sur la base d'un écart de la trajectoire d'un mouvement d'objet reconstruite à la trajectoire (10) de mouvement d'objet d'entraînement ; et
- on adapte des paramètres de réseau de l'autocodeur (9) ou de l'autocodeur (9) variationnel en fonction d'un résultat de l'évaluation de la fonction de perte.

6. Procédé suivant l'une des revendications 1 à 4, dans lequel l'algorithme (12) entraîné est conformé en module générateur d'un réseau GAN.

**7.** Procédé suivant la revendication 6, dans lequel

- pour la production de l'algorithme (12) entraîné on produit une pluralité de trajectoires (10) de mouvement d'objet d'entraînement, qui décrivent chacune un mouvement réel d'un objet de test ;
- pour chacune des trajectoires (10) de mouvement d'objet d'entraînement, on produit un vecteur d'entraînement dans l'espace (13) latent, en appliquant un réseau neuronal artificiel conformé comme codeur à la trajectoire (10) de mouvement d'objet d'entraînement respective ;
- on entraîne le module générateur et un module discriminateur du réseau GAN en utilisant les vecteurs d'entraînement comme données d'entrée.

**8.** Procédé suivant l'une des revendications 5 ou 7, dans lequel on contrôle au moyen d'un appareil photographique pour chacune des trajectoires (10) de mouvement d'objet d'entraînement pour la production de la trajectoire (10) de mouvement d'objet respective, l'objet de test pendant un laps de temps de test correspondant et on détermine la trajectoire (10) de mouvement d'objet d'entraînement respective sur la base d'images d'appareil photographique produites pendant le contrôle au moyen du au moins un appareil photographique.

**9.** Procédé suivant la revendication 8, dans lequel on appose sur l'objet (6) de test au moins un repère visuel et on détermine la trajectoire (10) de mouvement d'objet d'entraînement respective sur la base de positions correspondantes du au moins un repère visuel dans les images photographiques.

**10.** Procédé suivant l'une des revendications précédentes, dans lequel pour chaque stade d'optimisation

- on produit une reconstruction d'image présente en fonction de la pluralité d'images de projection en supposant que l'objet (6) s'est, pendant le déplacement du procédé d'imagerie, mis en mouvement suivant la trajectoire de mouvement d'objet présent, et on calcule la valeur présente de la fonction de coût en fonction de la reconstruction d'image présente ; ou
- on calcule la valeur présente de la fonction de coût en fonction d'un ensemble donné à l'avance de conditions de cohérence concernant la pluralité d'images de projection.

**11.** Procédé d'imagerie à base de rayons X, dans lequel on produit une pluralité d'images de projection d'un objet (6) et on effectue, sur la base de la pluralité d'images de projection, un procédé de production d'une reconstruction (14) d'image à compensation de mouvement suivant l'une des revendications précédentes.

**12.** Procédé suivant la revendication 11, dans lequel le procédé est un procédé de tomodensitométrie assisté par ordinateur à faisceau conique.

**13.** Système (7) de traitement de données, comprenant au moins une unité (7) informatique, qui est propre à effectuer un procédé de production d'une reconstruction (14) d'image à compensation de mouvement suivant l'une des revendications 1 à 10 précédentes.

**14.** Installation (1) d'imagerie sur la base des rayons X comportant une modalité (2) d'imagerie à rayons X, qui est agencée pour produire une pluralité d'images de projection d'un objet (6), un appareil de mémoire, qui met un algorithme (12) entraîné en mémoire, qui est entraîné pour affecter, à un vecteur latent d'un espace (13) latent donné à l'avance, une trajectoire de mouvement d'objet, ainsi qu'au moins une unité (7) informatique, qui est agencée pour,

- déterminer une trajectoire (10') de mouvement d'objet optimale en appliquant l'algorithme (12) entraîné à un vecteur latent optimum et produire la reconstruction (14) d'image à compensation de mouvement en fonction de la pluralité d'images de projection en supposant que l'objet (6) s'est, pendant l'exécution du procédé d'imagerie, mis en mouvement suivant la trajectoire (10') optimale de mouvement d'objet ; et
- minimiser une fonction de coût donnée à l'avance en utilisant le vecteur latent comme paramètre d'optimisation, afin de déterminer le vecteur latent optimum et déterminer ainsi pour chaque stade d'optimisation, par un vecteur latent présent en appliquant l'algorithme (12) entraîné au vecteur latent présent, une trajectoire de mouvement d'objet présent et calculer une valeur présente de la fonction de coût, en fonction de la pluralité d'images de projection en supposant que l'objet (6) s'est, pendant l'exécution du procédé d'imagerie, mis en mouvement suivant la trajectoire de mise en mouvement d'objet présente.

**15.** Produit de programme d'ordinateur comportant

- des premières instructions, qui lors de l'exécution par un système (7) de traitement de données, font que le système (7) de traitement de données exécute un procédé suivant l'une des revendications 1 à 10 ; et/ou
- des deuxièmes instructions, qui lors de l'exécution par un système (1) suivant la revendication (14), font que le système (1) exécute un procédé suivant l'une des revendications 1 à 12.

FIG 1

FIG 2

## FIG 3

## FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **J.WANG** ; **R. SCHAFFERT** ; **A. BORSDORF** ; **B. HEIGL** ; **X. HUANG** ; **J. HORNEGGER** ; **A. MAIER**. Dynamic 2-D/3-D rigid registration framework using point-to-plane correspondence model. *IEEE Transactions on Medical Imaging*, 2017, vol. 36 (9), 1939-1954
- **A. PREUHS** ; **M. MANHART** ; **A. MAIER**. Fast Epipolar Consistency without the Need for Pseudo Matrix Inverses. *Proc. 15th International Conference on Image Formation in X-Ray Computed Tomography*, 2018, 202-205

- **A. PREUHS** ; **M. MANHART** ; **E. HOPPE** ; **M. KOWARSCHIK** ; **A. MAIER**. Motion gradients for epipolar consistency. *Proc. 15th International Meeting on Fully Three-Dimensional Image Reconstruction in Radiology and Nuclear Medicine*, 2019, 110720D
- **A. PREUHS** ; **M. MANHART** ; **P. ROSER** ; **E. HOPPE** ; **H. YIXING** ; **M. PSYCHOGIOS** ; **M. KOWARSCHIK** ; **A. MAIER**. Appearance Learning for Image-based Motion Estimation in Tomography. *IEEE Transactions on Medical Imaging*, 2020, 1-10
- **V. SITZMANN** ; **J. MARTEL** ; **A. BERGMAN** ; **A. LINDELL** ; **G. WETZSTEIN**. Implicit neural representations with periodic activation functions. *Advances in Neural Information Processing Systems*, 2020, vol. 33, 7462-73